# EUROPÄISCHE PATENTSCHRIFT

(11) **EP 2 949 291 B1**
(45) Veröffentlichungstag und Bekanntmachung des Hinweises auf die Patenterteilung: **05.02.2020**
(21) Anmeldenummer: 14195866.0
(22) Anmeldetag: 02.12.2014
(51) Int. Cl.: A61F 2/16

(54) **Intraokularlinse und Mittel zum Herstellen einer Verbindung zwischen einer Intraokularlinse und einem Kapselsack eines Auges**
Intraocular lens and means for the production of a connection between an intraocular lens and a capsular bag of an eye
Lentille intraoculaire et moyen de fabrication d'une liaison entre une lentille intraoculaire et un sac capsulaire d'un oeil

(30) Priorität: 26.05.2014 DE 102014007533
(43) Veröffentlichungstag der Anmeldung: 02.12.2015
(73) Patentinhaber: Kirsch, Hinnerk, 22397 Hamburg (DE)
(72) Erfinder: Kirsch, Hinnerk, 22397 Hamburg (DE)
(74) Vertreter: RGTH

(56) Entgegenhaltungen:
- WO-A2-2008/023379
- WO-A2-2011/062826
- GB-A- 2 153 688
- US-A- 4 856 511
- US-A1- 2013 331 937

## Beschreibung

### Stand der Technik und Aufgabe

Die Erfindung betrifft eine Intraokularlinse. Ferner umfasst die Erfindung Mittel zur Herstellung einer lösbaren Verbindung zwischen einer Intraokularlinse und einem Kapselsack eines Auges.

Intraokularlinsen sind aus dem Stand der Technik grundsätzlich bekannt. Eine Intraokularlinse stellt eine künstliche Linse dar, die typischerweise in das Auge eines Menschen implantiert wird, wenn es entsprechende medizinische Indikationen dafür gibt, die natürliche Linse des Menschen auszutauschen. Die bekannteste Indikation ist der graue Star, bei der der sich die natürliche Linse des Menschen derart getrübt hat, dass die Sehfähigkeit beeinträchtigt wird.

Bei dem Einsetzen einer Intraokularlinse ergeben sich jedoch unterschiedliche Probleme, die die Ausrichtung der Intraokularlinse im Kapselsack betreffen. Die Intraokularlinse wird im Wesentlichen durch entsprechende Halteelemente in einem Randbereich der Intraokularlinse innerhalb des Kapselsacks gehalten. Dennoch garantieren die Haltelemente nur einen groben Sitz der Linse innerhalb des Kapselsacks. Die Intraokularlinse kann sich trotz der Haltelemente, die die Linse in radial äußerer Richtung abstützen, verschieben. So kann eine Deplatzierung der Intraokularlinse innerhalb des Kapselsacks erfolgen, bspw. eine lineare Verschiebung in der Sagittalebene des Auges oder auch in der Transversalebene. Ferner kann die Intraokularlinse auch verkippen sowie innerhalb des Kapselsacks rotieren. Eine Rotation wäre insbesondere im Falle von torischen Intraokularlinsen nachteilig, da hinsichtlich des zu korrigierenden Astigmatismus eine exakte Ausrichtung der torischen Achse der Intraokularlinse zur optischen Achse des Auges entscheidend ist. Neben der Tatsache, dass sich somit nachträgliche Deplatzierungen der Intraokularlinse ergeben können, erweist es sich bei Intraokularlinsen, vor allem bei torischen Intraokularlinsen, schon beim Einsetzen dieser als äußerst schwierig, die Intraokularlinse, insbesondere hinsichtlich der den Astigmatismus berücksichtigen torischen Achse, passend einzusetzen.

Die oben beschriebenen Deplatzierungen der Intraokularlinse, die entweder nachträglich erfolgen oder bereits beim Einsetzen dieser vorhanden sind, lösen Refraktionsfehler beim Patienten aus, die im ungünstigsten Fall sogar eine Korrektionsoperation notwendig machen können.

Die US 2013/0331937 A1 offenbart ein Intraokulares Implantat zur Verwendung in einem chirurgischen Verfahren, wie beispielsweise einer Kataraktoperation. Dieses Implantat hat einen Hauptbereich und einen peripheren Bereich. Der Hauptbereich ist plattenartig ausgebildet und kann eine Linse sein. Das Implantat hat zwei oder mehr Nasen, die vom peripheren Bereich in einer im Wesentlichen rechtwinkligen Richtung in Bezug auf die Ebene des Hauptbereichs vorstehen. In einem Verfahren zur Befestigung des Implantats im Auge ist vorgesehen, zwei oder mehrere Ausnehmungen in der Kapselwand vorzunehmen, wobei die Nasen in die Ausnehmungen eingebracht werden.

Die WO 2008/023379 A2 offenbart Implantationsausrüstung für Intraokularlinsen zur Verwendung beim Implantieren einer Intraokularlinse in einem menschlichen Auge. Die Ausrüstung umfasst ein pinzettenartiges Einsatzwerkzeug zum Einklemmen der Linse zwischen ihren vorderen und hinteren Oberflächen sowie ein hakenartiges Einsatzwerkzeug zur Unterstützung beim Fixieren der Haptik der Linse.

Die US 4,856,511 A umfasst ein Instrument zum Führen einer Haptik einer Linse in den Kapselsack. Das Instrument umfasst einen Edelstahlschaft mit einer Öse an einem Ende und einem Griff am anderen Ende.

WO 2011/062826 A2 offenbart eine Intraokularlinse mit einer verstellbaren Optik.

Es ist Aufgabe der vorliegenden Erfindung eine Intraokularlinse bzw. Mittel zum Einsetzen einer Intraokularlinse derart zu verbessern, dass eine möglichst exakte und verrutschungssichere Ausrichtung der Intraokularlinse erreicht wird.

### Darstellung der Erfindung: Aufgabe, Lösung, Vorteile

Gelöst wird diese Aufgabe durch eine erfindungsgemäße Intraokularlinse und erfindungsgemäßen Mitteln zur Herstellung einer lösbaren Verbindung zwischen einem Vorsprung einer Intraokularlinse mit einer korrespondierenden Ausnehmung in einem Kapselsack eines Auges.

Gemäß der Erfindung weist die Intraokularlinse eine Fixiervorrichtung zum Fixieren der Intraokularlinse in einem Kapselsack eines Auges auf, wobei die Fixiervorrichtung einen Vorsprung umfasst, wobei der Vorsprung eine erstes, freies Ende, ein zweites, der Intraokularlinse zugewandtes Ende und einen dazwischen liegenden Mittelbereich aufweist, wobei der Vorsprung radialsymmetrisch ausgebildet ist, wobei sich der Vorsprung ausgehend vom zweiten Ende im Mittelbereich verjüngt und sich in Richtung des ersten Endes erneut erweitert, wobei der Durchmesser des Vorsprungs im Mittelbereich kleiner ist als der Durchmesser am ersten Ende und der Durchmesser am zweiten Ende, und wobei der Durchmesser des Vorsprungs am ersten Ende größer ist als der Durchmesser am zweiten Ende.

Unter einer Intraokularlinse ist eine künstliche Linse zu verstehen, die insbesondere als Ersatz der natürlichen Linse des Auges eingesetzt wird. Phake Intraokularlinsen, die zusätzlich zur natürlichen Linse implantiert werden, sind bevorzugterweise ausgeschlossen.

Erfindungsgemäß weist die Intraokularlinse eine Fixiervorrichtung auf. Die Fixiervorrichtung umfasst einen Vorsprung, der dazu ausgebildet ist, die Intraokularlinse im Auge zu fixieren. Unter einem Vorsprung ist jede Art von Vorstand zu verstehen, der von der Intraokularlinse emporsteht und sich insbesondere durch sein Hervorstehen und seine Form von dem Rest der Intraokularlinse abhebt.

Bevorzugterweise ist der Vorsprung stiftartig ausgebildet. Somit ragt der Vorsprung insbesondere im Wesentlichen senkrecht von der Intraokularlinse hervor. Ferner bevorzugt ist der Vorsprung als Ausstülpung ausgebildet. Dabei ist der Vorsprung vorteilhafterweise aus demselben Material wie die restliche Intraokularlinse gebildet. Ferner kann der Vorsprung oder auch nur ein Teil des Vorsprungs aus einem anderen Material bestehen. Weiterhin bevorzugt ist der Vorsprung als Knopf ausgebildet. Somit ist die Intraokularlinse knopfmodifiziert ausgebildet um eine exaktere, verrutschungssichere und rotationstabile Platzierung der Intraokularlinse zu ermöglichen.

Insbesondere weist die Fixiervorrichtung mindestens einen Vorsprung auf. Ferner bevorzugt weist die Fixiervorrichtung genau einen Vorsprung auf. Die Fixiervorrichtung kann ebenso mehrere Vorsprünge aufweisen, die insbesondere in dieselbe Richtung der Intraokularlinse von dieser hervorstehen.

Bevorzugterweise ist die Fixiervorrichtung derart ausgebildet ist, dass sie zum Herstellen einer lösbaren Verbindung mit einer korrespondierenden Ausnehmung in dem Kapselsack des Auges geeignet ist.

Unter Fixieren ist die Befestigung der Intraokularlinse an mindestens einer Stelle, insbesondere am Kapselsack eines Auges, gemeint. Dabei bedeutet Befestigung nicht, dass sich die Intraokularlinse nicht auch noch gegenüber dem Befestigungspunkt, bspw. durch Rotation oder durch eine Translation aufgrund eines Spiels der Verbindung, bewegen könnte. Doch ist die Bewegungsfreiheit der Intraokularlinse aufgrund der durch die Fixiervorrichtung hergestellten Verbindung wesentlich reduziert.

Dabei ist die Ausnehmung in dem Kapselsack als Halteelement des Vorsprungs bei hergestellter Verbindung ausgebildet. Insbesondere ist die Ausnehmung als Öffnung im Kapselsack, bevorzugterweise als Knopfloch, ausgebildet. Die Öffnung bildet einen Durchbruch von einem Inneren des Kapselsacks zu einem Äußeren des Kapselsacks.

Die Verbindung zwischen Vorsprung der Intraokularlinse und der Ausnehmung des Kapselsacks wird bevorzugterweise durch Formschluss hergestellt. Insbesondere wird zur Herstellung der Verbindung zumindest ein freies Ende des Vorsprungs in die Ausnehmung eingeführt, bevorzugterweise durch die als Öffnung ausgebildete Ausnehmung von einem Inneren des Kapselsacks zu einem Äußeren des Kapselsacks durchgeführt. Dabei greifen die Ausnehmung und der Vorsprung bevorzugterweise ineinander und schaffen somit eine lösbare Verbindung der Intraokularlinse und des Kapselsacks.

Die Ausnehmung ist bevorzugterweise kreisrund ausgebildet. Bevorzugterweise beträgt der Durchmesser der Ausnehmung zwischen 300 µm und 700 µm, ferner bevorzugt zwischen 400 µm bis 600 µm, besonders bevorzugt zwischen 450 µm bis 550 µm, am meisten bevorzugt 500 µm.

Vorteilhaft bei einer derart ausgebildeten Intraokularlinse ist, dass die Fixiervorrichtung, insbesondere durch Herstellung der lösbaren Verbindung, ein positionsgenaues, exakteres und verrutschungssicheres Befestigen der Intraokularlinse in Bezug auf den Kapselsack und somit der Achse des Auges erlaubt. Deplatzierungen, unter anderem Dezentrierungen, bspw. ein lineares Verschieben der Intraokularlinse in der Transversalebene oder Sagittalebene, Verkippungen oder Rotationen der Intraokularlinse werden durch die Herstellung der lösbaren Verbindung im Wesentlichen unterdrückt.

Bevorzugterweise umfasst die Intraokularlinse einen optischen Bereich und einen Randbereich, wobei die Fixiervorrichtung, insbesondere der Vorsprung, im Randbereich der Intraokularlinse angeordnet ist. Je nach Ausgestaltung des jeweiligen Linsentyps kann die Fixiervorrichtung jedoch grundsätzlich auch im optischen Bereich vorgesehen sein.

Dabei ist der optische Bereich insbesondere als optische Linse ausgebildet. Er bildet vor allem den optisch wirksamen Teil der Intraokularlinse, der zur Korrektur von Refraktionsfehlern eines Auges, insbesondere aufgrund des Fehlens der natürlichen Linse, dient.

Der Randbereich wird typischerweise als "haptischer" Bereich bezeichnet. Dabei kann der Randbereich jegliche Art von Haptik aufweisen. Beispielsweise kann der Randbereich als Plattenhaptik, C-Haptik oder Z-Haptik ausgebildet sein. Der Randbereich schließt sich bevorzugterweise an den peripheren, also den radial äußeren, Rand des optischen Bereichs an.

Bevorzugterweise schließt sich der Randbereich an mindestens einen Abschnitt, bevorzugterweise an zwei gegenüberliegenden Abschnitten, des peripheren Randes des optischen Bereichs an diesen an. Der Randbereich kann sich ebenso in gesamter Umfangsrichtung an den optischen Bereich anschließen.

Insbesondere ist die Fixiervorrichtung, bevorzugterweise der Vorsprung, in einem radial inneren Bereich des Randbereichs angeordnet. Vorteilhafterweise ist der Abstand zwischen dem Vorsprung und dem optischen Bereich, insbesondere dem radial äußeren Rand des optischen Bereichs, kleiner als 1000 µm , ferner bevorzugt kleiner als 800 µm , besonders bevorzugt kleiner als 600 µm, ausgebildet.

Der Randbereich weist bevorzugterweise mindestens ein Halteelement auf, wobei der Vorsprung auf dem mindestens einen Haltelement angeordnet ist.

Die Halteelemente können je nach Haptik unterschiedlich ausgebildet sein. So können die Haltelemente als bevorzugterweise plattenförmige Abschnitte ausgebildet sein. Ferner können die Haltelemente als bevorzugterweise gebogen ausgebildete Haltearme ausgebildet sein. Diese Haltearme sind vorteilhafterweise an zwei gegenüberliegenden Abschnitten des optischen Bereichs bzw. des peripheren Randes des optischen Bereich, angeordnet. Dabei erstreckt sich deren gebogener Verlauf insbesondere zunächst in radial äußerer Richtung und anschließend zunehmend in Umfangsrichtung der Intraokularlinse.

Die Halteelemente können an zwei gegenüberliegenden peripheren Abschnitten des optischen Bereichs, insbesondere des radial äußeren Randes des optischen Bereichs, angeordnet sein. Der Abstand der jeweiligen Mitten der Haltelemente beträgt somit 180° in Umfangsrichtung der Intraokularlinse. Ebenso können die Haltelemente an drei oder vier peripheren Abschnitten des optischen Bereichs, insbesondere des radial äußeren Randes des optischen Bereichs, angeordnet sein, sodass der Abstand der Mitten zweier benachbarter Halteelemente in Umfangsrichtung 120° oder 90° beträgt.

Die Haltelemente dienen dazu der Intraokularlinse einen groben Sitz im Kapselsack zu ermöglichen, garantieren jedoch keine verrutschungssichere Platzierung so wie dies durch die Fixiervorrichtung erreicht wird. Der radial äußere Rand des Randbereichs ist bevorzugterweise als Anlagefläche am Kapselsack ausgebildet. Dabei stützen die Haltelemente die Intraokularlinse in radial äußere Richtung der Intraokularlinse im Kapselsack ab. In anderen Worten liegt der radial äußerste Bereich der Intraokularlinse mittels der Haltelemente am Kapselsack an.

Bevorzugterweise erstreckt sich die Fixiervorrichtung, insbesondere der mindestens eine Vorsprung, im Wesentlichen in Dickenrichtung der Intraokularlinse. Die Dickenrichtung der Intraokularlinse ist vorteilhafterweise im Wesentlichen parallel zu einer optischen Achse des optischen Bereichs der Intraokularlinse ausgebildet. Insbesondere ist die Dickenrichtung im Wesentlichen senkrecht zu einer radialen Richtung der Intraokularlinse ausgerichtet.

Bei der Erstreckung der Fixiervorrichtung können Abweichungen zur Dickenrichtung auftreten, dadurch dass sich die Intraokularlinse in Radialrichtung verjüngt. Besonders bevorzugt erstreckt sich die Fixiervorrichtung, insbesondere der mindestens eine Vorsprung, in der Richtung einer Normalen der Intraokularlinse an der Stelle des Vorsprungs.

Insbesondere weist die Intraokularlinse mindestens eine flächige Seite auf, wobei der Vorsprung von der Seite der Intraokularlinse in Dickenrichtung der Intraokularlinse oder in Normalenrichtung hervorragt. Dabei ist die flächige Seite dazu ausgebildet im implantierten Zustand der Intraokularlinse in Richtung Hornhaut des Auges und bevorzugterweise im Wesentlichen senkrecht zur optischen Achse des Auges ausgerichtet zu werden. Somit weist der Fixiervorrichtung im implantierten Zustand in Richtung Hornhaut des Auges.

In dem Falle, dass die Fixiervorrichtung mehrere Vorsprünge aufweist, stehen diese jeweils von derselben Seite der Intraokularlinse hervor. Sie erstrecken sich somit bevorzugterweise jeweils in Normalrichtung, weisen jedoch alle im Wesentlichen in dieselbe Richtung, insbesondere in Richtung Hornhaut im implantierten Zustand der Intraokularlinse.

Die Fixiervorrichtung ist bevorzugterweise zusätzlich zu den Haltelementen vorgesehen. Durch die Ausbildung des Vorsprungs in Dickenrichtung bzw. Normalenrichtung wird die Intraokularlinse durch die Fixiervorrichtung nicht in radial äußerer Richtung, sondern in Dickenrichtung oder Normalenrichtung, fixiert. Es erfolgt somit eine Fixierung in eine Richtung, die im Wesentlichen senkrecht zu der Richtung steht, in die die Haltearme die Intraokularlinse halten. Dies ermöglicht eine weitaus präzisere und verrutschungssichere Fixierung, als sie durch die Haltelemente erreicht wird.

Der Vorsprung ist radialsymmetrisch und im Querschnitt kreisrund ausgebildet. Insbesondere weist der Vorsprung eine Höhe auf, die sich in Dickenrichtung oder Normalenrichtung der Intraokularlinse erstreckt. In einer Richtung senkrecht zur Höhe bzw. senkrecht zur Dickenrichtung oder Normalenrichtung der Intraokularlinse weist der Vorsprung einen Durchmesser auf. Dabei ändert sich der Durchmesser entlang der Höhe der Intraokularlinse.

Vorteilhafterweise ist der Vorsprung der Intraokularlinse hinterschnitten ausgebildet. Bevorzugterweise ist der Vorsprung in einem Schnitt des Vorsprungs entlang der Höhe des Vorsprungs hinterschnitten ausgebildet.

Der Vorsprung weist ein erstes, freies Ende, ein zweites der Intraokularlinse zugewandtes Ende, sowie einen dazwischen liegenden Mittelbereich auf.

Der Durchmesser des Vorsprungs am zweiten Ende und/oder im Mittelbereich sind kleiner als der Durchmesser am ersten Ende. Der Durchmesser des Vorsprungs im Mittelbereich ist kleiner als der Durchmesser am ersten Ende und der Durchmesser am zweiten Ende, wobei der Durchmesser des Vorsprungs am ersten Ende größer ist als am zweiten Ende.

Insbesondere ist der Durchmesser des Vorsprungs derart gewählt, dass er am zweiten Ende und/oder im Mittelbereich dem Durchmesser der Ausnehmung im Kapselsack entspricht.

Ausgehend vom zweiten Ende des Vorsprungs verjüngt sich der Vorsprung in einem Mittelbereich des Vorsprungs und erweitert sich in Richtung des ersten Endes erneut. Bevorzugterweise ist der Vorsprung am ersten freien Ende gerundet ausgebildet, sodass der Bereich des Vorsprung an seinem ersten Ende bevorzugterweise schirmförmig ausgebildet ist, wobei der Mittelbereich des Vorsprungs halsförmig ausgebildet ist.

Durch die bevorzugte hinterschnittene Ausbildung des Vorsprungs wird gewährleistet, dass sich die lösbare Verbindung zwischen Vorsprung der Intraokularlinse und der Ausnehmung im Kapselsack nicht ohne äußeres Zutun wieder löst, sondern nur durch Ausüben einer entsprechenden externen Kraft.

In einer vorteilhaften Weiterbildung sind die Intraokularlinse, insbesondere der optische Bereich und der Randbereich, und die Fixiervorrichtung einstückig ausgebildet. Alternativ können die Intraokularlinse und der Vorsprung auch mehrstückig ausgebildet sein. So kann der Vorsprung ein separater Teil zum Randbereich und optischen Bereich der Intraokularlinse darstellen, der dazu ausgebildet ist mit der Intraokularlinse verbunden werden zu können, insbesondere durch eine Steck- oder Schraubverbindung. Ferner bevorzugt kann ein Teil des Vorsprungs, bevorzugterweise ein der Intraokularlinse abgewandter Teil, separat ausgebildet sein, jedoch derart, dass dieser mit einem weiteren an dem Randbereich des Intraokularlinse befestigten Teil des Vorsprungs verbunden werden kann.

Vorteilhafterweise ist die Intraokularlinse torisch ausgebildet. Insbesondere ist die Intraokularlinse, bevorzugterweise deren optischer Bereich, dazu ausgebildet, einen Astigmatismus eines Auges zu korrigieren. Vorteilhafterweise ist die Intraokularlinse, insbesondere deren optischer Bereich, dazu asphärisch ausgebildet und weist bevorzugterweise eine torische Achse auf, die zur Korrektur des Astigmatismus auf eine entsprechende Achse des Auges ausgerichtet werden muss. Eine derartige Ausrichtung beim Einsetzen der Intraokularlinse ist sehr schwierig. Ebenso ist eine nachträgliche geringe Rotation der Intraokularlinse möglich, die in einer weitaus schlechteren Korrektur des Astigmatismus resultiert. Durch die erfindungsgemäße Ausgestaltung der Intraokularlinse ist eine Fixierung der Intraokularlinse möglich, die eine exakte Ausrichtung der Intraokularlinse garantiert.

Ferner umfasst die Erfindung ein Mittel zur Herstellung einer lösbaren Verbindung zwischen einem Vorsprung einer erfindungsgemäßen Intraokularlinse mit einer korrespondierenden Ausnehmung in einem Kapselsack eines Auges, wobei das Mittel zum Halten der Intraokularlinse ausgebildet ist, wobei das Mittel an einem ersten Ende einen Griffbereich zum Halten des Mittels aufweist, wobei das Mittel an einem zweiten Ende einen Bereich zum Halten der Intraokularlinse aufweist, wobei das Mittel zwischen dem ersten Ende und dem zweiten Ende derart ausgebildet ist, dass sich das Mittel ausgehend vom ersten Ende in einem ersten Bereich im Wesentlichen entlang einer ersten Gerade erstreckt und dass sich das Mittel in einem zweiten Bereich im Wesentlichen entlang einer zweiten Gerade erstreckt, und wobei die erste Gerade und die zweite Gerade in einem Winkel zueinander stehen. Der Winkel beträgt vorteilhaft zwischen 15° und 75°, bevorzugt zwischen 20° und 70°, besonders bevorzugt zwischen 30° und 60° und ganz besonders bevorzugt zwischen 40° und 50°. Dabei schließen die erste Gerade und die zweite Gerade genau genommen zwei Winkel ein. Bei obigen Winkelbereich ist der kleinere, unter 90° liegende Winkel gemeint, und nicht der komplementäre Winkel, der größer als 90° beträgt.

Ferner bevorzugt weist das Mittel in einem Übergangsbereich zwischen dem ersten und dem zweiten Bereich mindestens einen, bevorzugterweise genau einen, Knick und/oder eine Krümmung, auf. Dabei ist der Übergangsbereich. zwischen dem ersten Ende des Mittels und dem zweiten Ende des Mittels ausgebildet. Bevorzugt ist der Übergangsbereich im Bereich zwischen 2/5 bis einschließlich 5/5 der Länge des Mittels, ausgehend vom ersten Ende des Mittels, ausgebildet. Besonders bevorzugt ist der Übergangsbereich im Bereich zwischen 3/5 bis einschließlich 4/5 der Länge des Mittels ausgebildet. Das Mittel erstreckt sich bevorzugterweise ausgehend von dem ersten Ende des Mittels entlang der ersten Gerade, woraufhin ein Knick in Richtung einer zweiten Gerade erfolgt, ab der sich das Mittel entlang der zweiten Gerade zum zweiten Ende hin erstreckt.

Erfindungsgemäß ist der Bereich zum Halten der Intraokularlinse gebogen, bevorzugterweise hakenförmig, ausgebildet. Die erste Gerade und die zweite Gerade definieren eine erste Ebene. Der Bereich zum Halten der Intraokularlinse erstreckt sich durch die gebogene Ausführung in einer zweiten Ebene, die dadurch definiert ist, dass sie unter einem Winkel, bevorzugt senkrecht, zu der ersten Ebene ausgebildet ist und die zweite Gerade umfasst.

Das oben beschriebene Mittel wird im Folgenden erstes Mittel genannt. Bevorzugterweise ist das erste Mittel als Intraokular-Haltespatel ausgebildet.

Ferner betrifft die Erfindung ein Mittel zur Herstellung einer lösbaren Verbindung zwischen einem Vorsprung einer erfindungsgemäßen Intraokularlinse mit einer korrespondierenden Ausnehmung in einem Kapselsack eines Auges, wobei das Mittel zum Einführen eines freien Endes des Vorsprungs in die Ausnehmung des Kapselsacks ausgebildet ist, wobei das Mittel an einem ersten Ende einen Griffbereich zum Halten des Mittels aufweist, wobei das Mittel an einem zweiten Ende einen Bereich zum Einführen des freien Endes des Vorsprungs in die Ausnehmung des Kapselsacks aufweist, wobei der Bereich zum Einführen eines freien Endes des Vorsprungs in die Ausnehmung des Kapselsacks ringförmig ausgebildet ist.

Unter dem Begriff Einführen eines freien Endes des Vorsprungs wird bevorzugterweise ein Durchführen zumindest des freien Endes des Vorsprungs durch die als Öffnung ausgebildete Ausnehmung verstanden.

Dabei ist die Ausnehmung in dem Kapselsack als Halteelement des Vorsprungs bei hergestellter Verbindung ausgebildet. Insbesondere ist die Ausnehmung als Öffnung im Kapselsack ausgebildet. Die Öffnung bildet einen Durchbruch von einem Inneren des Kapselsacks zu einem Äußeren des Kapselsacks. Bevorzugterweise ist das Mittel derart ausgebildet, dass der Vorsprung, zumindest dessen freies Ende, durch die als Öffnung ausgebildete Ausnehmung geführt wird. Dabei wird bevorzugterweise ebenso zumindest ein Teil des Mittelbereichs des Vorsprungs in die Ausnehmung eingeführt.

Erfindungsgemäß ist das Mittel zwischen dem ersten Ende und dem zweiten Ende derart ausgebildet, dass sich das Mittel ausgehend vom ersten Ende in einem ersten Bereich im Wesentlichen entlang einer ersten Gerade erstreckt und dass sich das Mittel in einem zweiten Bereich im Wesentlichen entlang einer zweiten Gerade erstreckt, und wobei die erste Gerade und die zweite Gerade in einem Winkel zueinander stehen. Der Winkel beträgt vorteilhaft zwischen 15° und 75°, bevorzugt zwischen 20° und 70°, besonders bevorzugt zwischen 30° und 60° und ganz besonders bevorzugt zwischen 40° und 50°. Dabei schließen die erste Gerade und die zweite Gerade genau genommen zwei Winkel ein. Bei obigen Winkelbereich ist der kleinere, unter 90° liegende Winkel gemeint, und nicht der komplementäre Winkel, der größer als 90° beträgt.

Ferner bevorzugt weist das Mittel in einem Übergangsbereich zwischen dem ersten und dem zweiten Bereich mindestens einen, bevorzugterweise genau einen, Knick und/oder eine Krümmung, auf. Dabei ist der Übergangsbereich zwischen dem ersten Ende des Mittels und dem zweiten Ende des Mittels ausgebildet. Bevorzugt ist der Übergangsbereich im Bereich zwischen 2/5 bis einschließlich 5/5 der Länge des Mittels, ausgehend vom ersten Ende des Mittels, ausgebildet. Besonders bevorzugt ist der Übergangsbereich im Bereich zwischen 3/5 bis einschließlich 4/5 der Länge des Mittels ausgebildet. Das Mittel erstreckt sich bevorzugterweise ausgehend von dem ersten Ende des Mittels entlang der ersten Gerade, woraufhin ein Knick in Richtung einer zweiten Gerade erfolgt, ab der sich das Mittel entlang der zweiten Gerade zum zweiten Ende hin erstreckt.. Dabei weist der ringförmige Bereich insbesondere eine Dickenrichtung auf, wobei die Dickenrichtung des ringförmigen Bereichs senkrecht zur zweiten Gerade, entlang derer sich der zweite Bereich des Mittels erstreckt, ausgebildet ist.

Erfindungsgemäß ist der Bereich zum Einführen eines freien Endes des Vorsprungs in die Ausnehmung des Kapselsacks kegelstumpfförmig ausgebildet. Hierbei verjüngt sich der Bereich bevorzugterweise in eine Richtung senkrecht zur zweiten Gerade, entlang derer sich der zweite Bereich des Mittels erstreckt.

Der kegelstumpfförmige Bereich zum Einführen des freien Endes des Vorsprungs in die Ausnehmung des Kapselsacks ist insbesondere derart ausgebildet, dass er lediglich die Mantelfläche eines Kegelstumpfes umfasst. Bevorzugterweise weist der kegelstumpfförmige Bereich zwei, insbesondere kreisrunde, Öffnungen auf. Eine erste Öffnung liegt bevorzugterweise auf der zweiten Gerade, entlang derer sich der zweite Teil des Mittels erstreckt. Eine zweite Öffnung liegt insbesondere in einem Abstand zur zweiten Geraden, der der Höhe des Kegelstumpfes bzw. des Bereichs zum Einführen des freien Endes des Vorsprungs in die Ausnehmung des Kapselsacks entspricht. Die erste Öffnung, insbesondere deren Durchmesser, ist bevorzugterweise größer ausgeführt als die zweite Öffnung, insbesondere deren Durchmesser. Die erste Öffnung ist vorteilhafterweise derart ausgebildet, dass deren Durchmesser dem größten Durchmesser des Vorsprungs des Intraokularlinse entspricht oder kleiner als dieser ist.

Das oben beschriebene Mittel wird im Folgenden als zweites Mittel bezeichnet und ist bevorzugterweise als Knopflöffel zum Überstülpen der Ausnehmung über den Vorsprungs ausgebildet.

Bevorzugterweise ist das erste Mittel zum Zusammenwirken mit einem zweiten Mittel zur Herstellung einer lösbaren Verbindung zwischen einem Vorsprung einer Intraokularlinse mit einer korrespondierenden Ausnehmung in einem Kapselsack eines Auges ausgebildet. Während mit dem zweiten Mittel ein gewisser Druck zum Einführen des freien Endes des Vorsprungs in die Ausnehmung erreicht werden kann, ist das erste Mittel derart korrespondierend zum zweiten Mittel ausgebildet, dass mit diesem die Intraokularlinse gehalten und somit der nötige Gegendruck auf die Intraokularlinse ausgeübt werden kann.

Dafür bilden das erste und das zweite Mittel insbesondere ein System, insbesondere ein System komplementärer Werkzeuge, zur Herstellung einer lösbaren Verbindung zwischen einem Vorsprung einer Intraokularlinse mit einer korrespondierenden Ausnehmung in einem Kapselsack eines Auges. Ferner bevorzugt bildet die erfindungsgemäße Intraokularlinse und das erste Mittel und/oder das zweite Mittel ein System.

Die Erfindung kann zum Durchführen eines Verfahrens zum Einsetzen und Fixieren einer einen Vorsprung aufweisenden Intraokularlinse in einem Kapselsack eines Auges dienen, umfassend die Schritte des Herstellens einer Ausnehmung im Kapselsack des Auges, des Einsetzens der Intraokularlinse im Kapselsack und des Herstellens einer lösbaren Verbindung zwischen dem Vorsprung der Intraokularlinse und der Ausnehmung im Kapselsack.

Insbesondere ist die Ausnehmung als Öffnung im Kapselsack ausgebildet. Das Verfahren umfasst somit die Herstellung einer Öffnung im Kapselsack. Insbesondere wird zur Herstellung der Ausnehmung bzw. Öffnung ein Femtosekundenlaser verwendet. Die Verwendung eines Femtosekundenlasers hat den Vorteil, dass dieser eine mikrometergenaue und in Größe und Platzierung vorhersehbare Herstellung der Ausnehmung erlaubt, die mit einer manuellen Herstellungstechnik nur bedingt möglich ist.

Dabei wird die Ausnehmung bevorzugterweise kreisrund hergestellt, insbesondere derart, dass deren Durchmesser kleiner ist als der größte Durchmesser des Vorsprungs. Insbesondere beträgt der Durchmesser der Ausnehmung zwischen 300 µm und 700 µm , ferner bevorzugt zwischen 400 µm bis 600 µm , besonders bevorzugt zwischen 450 µm bis 550 µm , am meisten bevorzugt 500 µm, wobei der größte Durchmesser des Vorsprungs insbesondere 400 µm bis 800 µm , bevorzugterweise 500 µm bis 700 µm, ferner bevorzugt 550 µm bis 650 µm, besonders bevorzugt 600 µm beträgt.

Das Einsetzen der Intraokularlinse im Kapselsack umfasst vorteilhafterweise die Herstellung einer weiteren Ausnehmung, bevorzugterweise Öffnung, im Kapselsack zum Einbringen der Intraokularlinse. Dabei ist die Ausnehmung zum Einbringen der Intraokularlinse ebenfalls bevorzugterweise kreisrund ausgebildet. Ferner ist die Ausnehmung zum Einbringen der Intraokularlinse wesentlich größer ausgeführt als die Ausnehmung zum Herstellen einer lösbaren Verbindung mit dem Vorsprung der Intraokularlinse. Auch die Ausnehmung zum Einbringen der Intraokularlinse wird insbesondere mittels eines Femtosekundenlasers hergestellt. Bevorzugterweise dient eine Pinzette dazu, den die Öffnung bedeckenden Bereich des Kapselsacks aufzuklappen um somit mittels der Ausnehmung bzw. Öffnung an das Innere des Kapselsacks zu gelangen.

Ferner umfasst der Schritt des Einsetzen der Intraokularlinse im Kapselsack bevorzugterweise das Entfernen einer natürlichen Linse des Auges oder einer künstlichen Linse des Auges. Das Entfernen einer natürlichen Linse des Auges umfasst insbesondere das Zerkleinern der natürlichen Linse mithilfe Ultraschall oder mithilfe eines Femtosekundenlasers und ein anschließendes Absaugen der zerkleinerten Linse.

Zudem umfasst der Schritt des Einsetzen der Intraokularlinse im Kapselsack bevorzugterweise das Einbringen der Intraokularlinse in das Innere des Kapselsacks sowie bevorzugterweise ein grobes Fixieren des Intraokularlinse in radial äußerer Richtung durch deren Halteelemente.

Die Ausnehmung zum Einbringen der Intraokularlinse und/oder die Ausnehmung zum Herstellen einer lösbaren Verbindung mit dem Vorsprung der Intraokularlinse werden bevorzugterweise in einem Bereich der Kapselsacks hergestellt, der in Richtung Hornhaut des Auges gewandt ist. Bevorzugterweise handelt es sich bei der Herstellung beider Öffnungen um eine Kapsulorhexis, bei der Herstellung der Ausnehmung zum Herstellen einer lösbaren Verbindung mit dem Vorsprung der Intraokularlinse insbesondere um eine Knopflochkapsulorhexis.

Vorteilhafterweise weist der Vorsprung ein freies Ende auf, wobei die Herstellung einer lösbaren Verbindung zwischen dem Vorsprung und der Ausnehmung im Kapselsack durch Einführen des freien Endes des Vorsprungs in die Ausnehmung des Kapselsacks erreicht wird.

Der Schritt umfasst ferner ein Ausrichten der Intraokularlinse derart, dass der Vorsprung der Intraokularlinse und die Ausnehmung im Kapselsack zum Herstellen einer lösbaren Verbindung mit dem Vorsprung der Intraokularlinse in unmittelbarer Nähe zueinander, insbesondere in Dickenrichtung der Intraokularlinse, angeordnet sind.

Insbesondere wird zum Einführen des freien Endes des Vorsprungs ein erfindungsgemäßes zweites Mittel verwendet, wobei bevorzugterweise zum Ausüben eines Gegendrucks ein erfindungsgemäßes erstes Mittel verwendet wird.

Dabei wird das zweite Mittel bevorzugterweise derart eingesetzt, dass es von außerhalb des Kapselsacks im Bereich der Ausnehmung zum Herstellen einer lösbaren Verbindung mit dem Vorsprung der Intraokularlinse auf den Kapselsack aufgesetzt wird. Das erste Mittel wird insbesondere derart eingesetzt, dass es die Intraokularlinse von der Gegenseite her hält bzw. stützt. Mithilfe des zweiten Mittels wird ein Druck auf den Kapselsack im Bereich der Ausnehmung zum Herstellen einer lösbaren Verbindung mit dem Vorsprung der Intraokularlinse ausgeübt. Um zu verhindern, dass sich die im Kapselsack befindliche Intraokularlinse bewegt, wird diese mittels des ersten Mittels gehalten. Aufgrund des Zusammenspiels der ersten und des zweiten Mittels kann demnach ein Druck auf die Ausnehmung des Kapselsacks ausgeübt werden und ein entsprechender Gegendruck auf Seiten der Intraokularlinse. Dadurch wird die Ausnehmung, deren Durchmesser bevorzugterweise kleiner ist als der größte Durchmesser des Vorsprungs, insbesondere entsprechend über den Vorsprung gestülpt um eine lösbare Verbindung herzustellen. Dabei kann die Ausnehmung zumindest temporär geweitet werden um über den größten Durchmesser des Vorsprungs zu passen.

Somit wird zumindest das freie Ende des Vorsprungs durch bevorzugterweise als Öffnung ausgebildete Ausnehmung von dem Inneren des Kapselsacks zum Äußeren des Kapselsacks durchgeführt.

### Bevorzugte Ausführungsformen der Erfindung

Die Erfindung wird nachstehend anhand der Zeichnungen beispielhaft näher erläutert. Es zeigen dabei in schematischer Darstellung:
- Figur 1 eine perspektivische Ansicht einer erfindungsgemäßen Intraokularlinse,
- Figur 2 eine Schnittdarstellung eines Teils des Randbereichs und des Vorsprungs der erfindungsgemäßen Intraokularlinse gemäß Figur 1 entlang der Höhe des Vorsprungs,
- Figur 3 eine perspektivische Ansicht eines erfindungsgemäßen ersten Mittels,
- Figur 4 eine perspektivische Ansicht eines erfindungsgemäßen zweiten Mittels, und
- Figuren 5 bis 8 in perspektivischer Ansicht verschiedene Schritte eines Verfahrens, das nicht Teil der Erfindung ist.

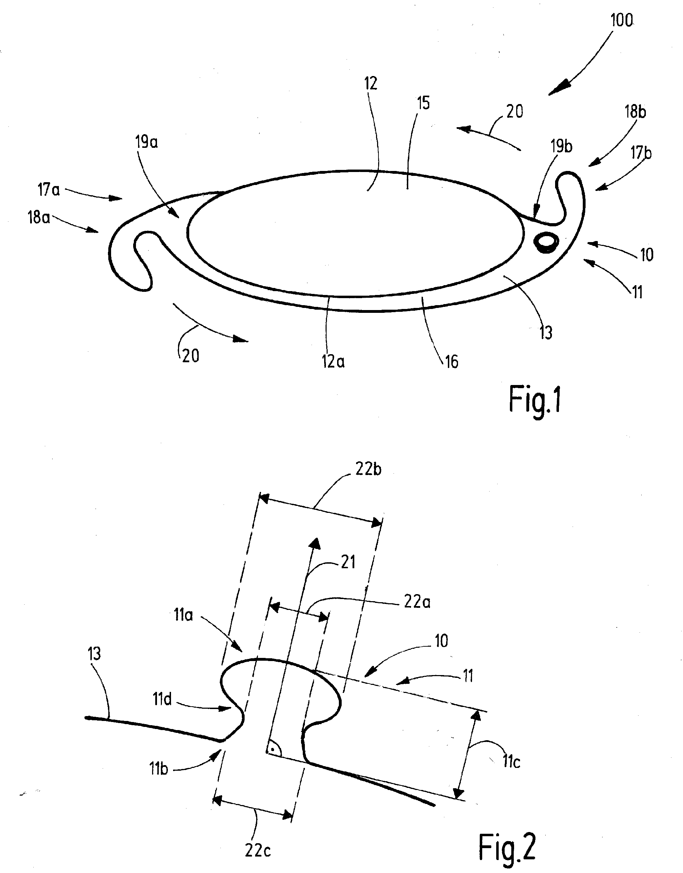

Figur 1 zeigt eine perspektivische Ansicht einer erfindungsgemäßen Intraokularlinse (100). Dabei weist die Intraokularlinse (100) einen optischen Bereich (12) und einen Randbereich (13) auf. Der optische Bereich (12) ist als optische Linse (15) ausgebildet. Der Randbereich (13) schließt sich an den radial äußeren Rand (12a) des optischen Bereichs (12) an.

Dabei umfasst der Randbereich (13) einen sich entlang der gesamten Umfangsrichtung (20) an den radial äußeren Rand (12a) anschließenden Steg (16). Ferner umfasst der Randbereich (12) zwei Halteelemente (17a, 17b), die in Form zweier Haltearme (18a, 18b) ausgebildet sind. Bei dem Randbereich (13) handelt es sich insgesamt um eine C-Haptik. Die Haltearme (18a, 18b) sind an zwei gegenüberliegenden Abschnitten (19a, 19b) des radial äußeren Randes (12a) des optischen Bereichs (12) angeordnet. Die Haltearme (18a, 18b) sind gebogen ausgebildet. Dabei erstrecken sich die Haltearme (18a, 18b) ausgehend von dem jeweiligen Abschnitt (19a, 19b) des radial äußeren Randes (12a) des optischen Bereichs (12) zunächst radial nach außen und krümmen sich anschließend zunehmend in eine Umfangsrichtung (20) der Intraokularlinse (100). Dabei sind die Haltearme (19a, 19b) in Umfangsrichtung (20) gleich ausgerichtet.

Auf dem Randbereich (13), insbesondere auf einem Haltearm (18b), ist die Fixiervorrichtung (10) angeordnet. Die Fixiervorrichtung (10) weist einen Vorsprung (11) auf, der von der Intraokularlinse (100), besonders dessen Randbereich (13), hervorsteht. Der Vorsprung (11) ist radialsymmetrisch ausgebildet und weist eine Höhe (11c) auf. Dabei erstreckt sich die Höhe (11c) des Vorsprungs (11) in eine Normalenrichtung (21) der Intraokularlinse (100) an der Stelle des Vorsprungs (11).

In Figur 2 ist eine Schnittdarstellung eines Teils des Randbereichs (13) und des Vorsprungs (11) der erfindungsgemäßen Intraokularlinse (100) gemäß Figur 1 entlang der Höhe (11c) des Vorsprungs (11) gezeigt. Der Vorsprung (11) und der Randbereich (13) sind aus demselben Material und einstückig ausgebildet.

Der Vorsprung (11) weist ein freies erstes Ende (11a) auf, das der Intraokularlinse (100) abgewandt ausgebildet ist, und ein zweites Ende (11b), das dem Randbereich (13) der Intraokularlinse (100) zugewandt ist. Dazwischen weist der Vorsprung (11) einen Mittelbereich (11d) auf.

Der Vorsprung (11) ist hinterschnitten ausgebildet. Der Durchmesser (22a) des Vorsprungs (11) in einem Querschnitt des Vorsprungs (11) in dem Mittelbereich (11d) des Vorsprungs (11) ist kleiner ausgebildet ist als der Durchmesser (22b) des Vorsprungs (11) am ersten freien Ende (11a) und der Durchmesser (22c) des Vorsprungs (11) am zweiten Ende (11b) des Vorsprungs (11). Ferner ist der Durchmesser (22c) am zweiten Ende (11b) des Vorsprungs (11) kleiner ausgebildet als der Durchmesser (22b) am ersten freien Ende (11a) des Vorsprungs (11). Somit verjüngt sich der Durchmesser des Vorsprungs (11) ausgehend vom zweiten Ende (11b), wobei der kleinste Durchmesser (22a) in einem Mittelbereich (11d) des Vorsprungs (11) angeordnet ist. Ausgehend vom Mittelbereich (11d) des Vorsprungs (11) erweitert sich der Durchmesser erneut in Richtung des zweiten Endes (11b) des Vorsprungs (11). Am ersten freien Ende (11a) des Vorsprungs (11) ist dieser gerundet ausgebildet.

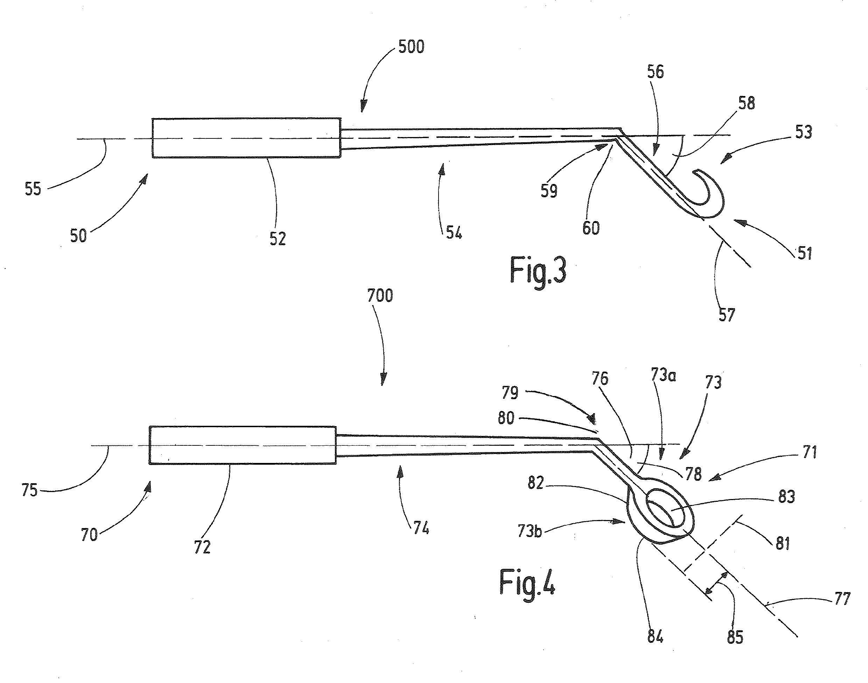

In Figur 3 ist eine perspektivische Ansicht eines erfindungsgemäßen Mittels (500) zur Herstellung einer lösbaren Verbindung zwischen einem Vorsprung (11) einer Intraokularlinse (100) mit einer korrespondierenden Ausnehmung (31) in einem Kapselsack (30) eines Auges (300) gezeigt, wobei das Mittel (500) zum Halten der Intraokularlinse (100) ausgebildet ist.

Das Mittel (500) weist ein erstes Ende (50) und ein zweites Ende (51) auf. An seinem ersten Ende (50) umfasst das Mittel (500) einen Griffbereich (52) zum Halten des Mittels (500).

Ferner umfasst das Mittel (500) einen ersten Bereich (54), einen zweiten Bereich (56) und einen Übergangsbereich (59) zwischen dem ersten Bereich (54) und dem zweiten Bereich (56). Ausgehend vom ersten Ende (50) erstreckt sich das Mittel (500) im ersten Bereich (54) entlang einer ersten Gerade (55). In dem Übergangsbereich (59) weist das Mittel (500) einen Knick (60) auf. Ausgehend von dem Knick (60) erstreckt sich das Mittel (500) in Richtung des zweiten Endes (51) entlang einer zweiten Gerade (57). Dabei weisen die erste Gerade (54) und die zweite Gerade (56) einen Winkel (58) zueinander auf.

Ferner umfasst das Mittel (500) an seinem zweiten Ende (51) einen Bereich (53) zum Halten der Intraokularlinse (100). Der Bereich (53) zum Halten der Intraokularlinse (100) ist gebogen, insbesondere hakenförmig, ausgebildet. Der bogenförmige Verlauf des Bereichs (53) zum Halten der Intraokularlinse (100) erstreckt sich in einer zweiten Ebene. Dabei ist die zweite Ebene dadurch definiert, dass sie die zweite Gerade (57) umfasst und sie unter einem Winkel, bevorzugt senkrecht, zu einer ersten Ebene ausgerichtet ist, wobei die erste Ebene durch die erste Gerade (55) und die zweite Gerade (57) definiert wird.

In der Darstellung von Fig. 3 ist der zweite Bereich 56 gegenüber dem ersten Bereich 54 nach unten abgeknickt wohin gegen der hakenförmige Bereich 53 nach oben und/oder seitlich vom zweiten Bereich 56 vorsteht.

In Figur 4 ist ein erfindungsgemäßes Mittel (700) zur Herstellung einer lösbaren Verbindung zwischen einem Vorsprung (11) einer Intraokularlinse (100) mit einer korrespondierenden Ausnehmung (31) in einem Kapselsack (30) eines Auges (300) dargestellt, wobei das Mittel (700) zum Einführen eines freien Endes (11a) des Vorsprungs (11) in die Ausnehmung (31) des Kapselsacks (30) ausgebildet ist.

Das Mittel (700) weist ein erstes Ende (70) und ein zweites Ende (71) auf. An dem ersten Ende (70) weist das Mittel (700) einen Griffbereich (72) zum Halten des Mittels (700) auf.

Ferner umfasst das Mittel (700) einen ersten Bereich (74), einen zweiten Bereich (76) und einen Übergangsbereich (79) zwischen dem ersten Bereich (74) und dem zweiten Bereich (76). Ausgehend vom ersten Ende (70) erstreckt sich das Mittel (700) im ersten Bereich (74) entlang einer ersten Gerade (75). In dem Übergangsbereich (79) weist das Mittel (700) einen Knick (80) auf. Ausgehend von dem Knick (80) erstreckt sich das Mittel (700) in Richtung des zweiten Endes (71) entlang einer zweiten Gerade (77). Dabei weisen die erste Gerade (74) und die zweite Gerade (76) einen Winkel (78) zueinander auf.

An dem zweiten Ende (71) umfasst das Mittel (700) einen Bereich (73) zum Einführen des freien Endes (11a) des Vorsprungs (11) in die Ausnehmung (31) des Kapselsacks (30).

Der Bereich (73) zum Einführen des freien Endes (11a) des Vorsprungs (11) in die Ausnehmung (31) des Kapselsacks (30) ist ringförmig, insbesondere kegelstumpfförmig, ausgebildet. Der Bereich (73) zum Einführen des freien Endes (11a) des Vorsprungs (11) in die Ausnehmung (31) des Kapselsacks (30) weist ein erstes Ende (73a), ein zweites Ende (73b) sowie eine Dickenrichtung (81) auf, die senkrecht zur zweiten Gerade (77) steht. Dabei verjüngt sich der Bereich (73) ausgehend vom ersten Ende (73a) entlang der Dickenrichtung (81) in Richtung des zweiten Endes (73b).

Der Bereich (73) zum Einführen des freien Endes (11a) des Vorsprungs (11) in die Ausnehmung (31) des Kapselsacks (30) wird durch einen Kegelstumpfmantel (82) gebildet und umfasst sowie eine erste Öffnung (83) am ersten Ende (73a) und eine zweite Öffnung (84) am zweiten Ende (73b). Die erste Öffnung (83) ist auf der zweiten Geraden (77), entlang derer sich das Mittel (700) erstreckt, angeordnet. Die zweite Öffnung (84) liegt insbesondere in einem Abstand zur zweiten Geraden (77), der der Höhe (85) des Kegelstumpfes bzw. des Bereichs (73) in Dickenrichtung (81) entspricht. Der Durchmesser der ersten Öffnung (83) ist größer ausgeführt als der Durchmesser der zweiten Öffnung (84).

In der Darstellung von Fig. 4 ist der zweite Bereich 76 gegenüber dem ersten Bereich 74 nach unten abgeknickt, wohingegen der Bereich (73) zum Einführen des freien Endes (11a) des Vorsprungs (11) in die Ausnehmung (31) des Kapselsacks (30) in Verlängerung des zweiten Bereichs 76 angeordnet ist.

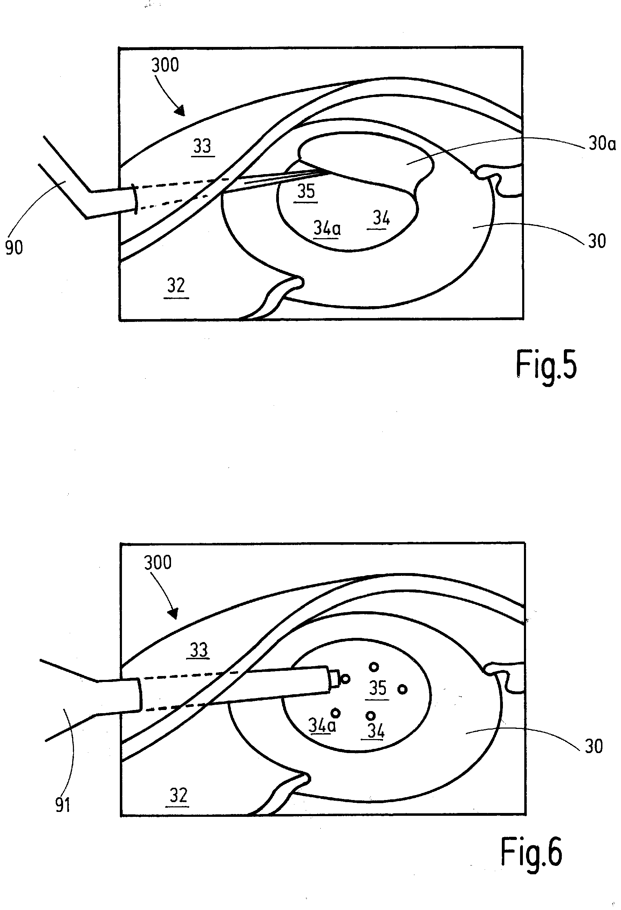

Die Figuren 5 bis 8 zeigen in perspektivischer Ansicht verschiedene Schritte eines Verfahrens, das nicht Teil der Erfindung ist.

In Figur 5 ist die perspektivische Ansicht eines Auges (300) gezeigt umfassend eine Hornhaut (33), eine Iris (32) sowie einen Kapselsack (30). Ferner weist der Kapselsack (30) eine Ausnehmung (34) zum Einbringen einer Intraokularlinse (100) auf. Die Ausnehmung (34) ist als Öffnung (34a) zum Einbringen der Intraokularlinse (100) ausgebildet. Die Öffnung (34a) wurde mittels eines Femtosekundenlasers hergestellt. Ein Bereich (30a) des Kapselsacks (30) bedeckt die zuvor hergestellte Öffnung (34a). In der Figur 5 ist zu sehen, wie mittels einer Pinzette (90) der die Öffnung (34a) bedeckende Bereich (30a) des Kapselsacks (30) aufgeklappt wird um somit mittels der Öffnung (34a) an das Innere des Kapselsacks (30) zu gelangen.

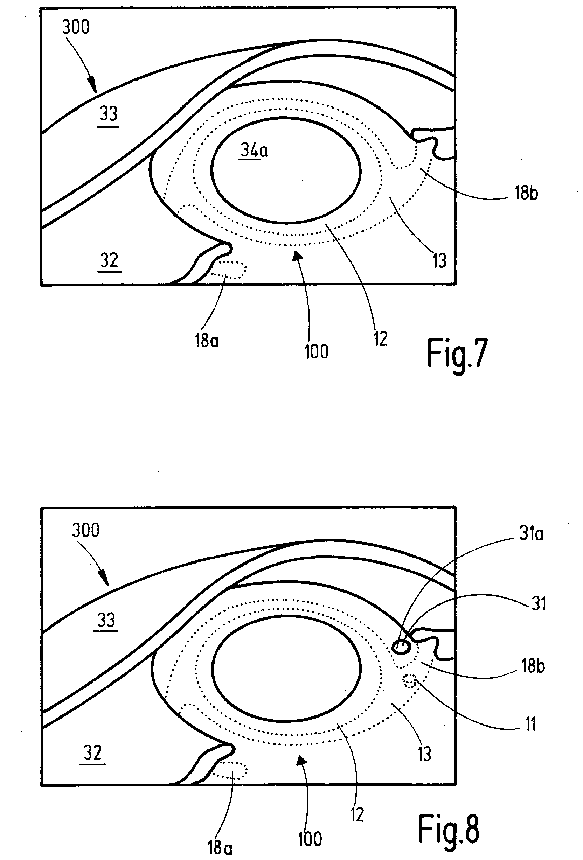

Figur 6 zeigt, wie die natürliche Augenlinse (35) des Auges (300) mittels einer Ultraschallsonde (91) zerkleinert wird. Im Anschluss wird die natürliche Augenlinse (35) abgesaugt. In Figur 7 ist die Intraokularlinse (100) im Kapselsack (30) dargestellt. Davor wurde die Intraokularlinse (100) durch die Öffnung (34a) zum Einbringen der Intraokularlinse (100) eingebracht und mittels der Haltearme (18a, 18b) festgeklemmt. Zum einfacheren Einbringen in den Kapselsack (30) ist die Intraokularlinse (100) typischerweise faltbar ausgebildet. Figur 8 zeigt, wie ein Vorsprung (11) der Intraokularlinse (100) mit einer Ausnehmung (31) zum Herstellen einer lösbaren Verbindung verbunden wird. Dabei ist die Ausnehmung (31) als Öffnung (31a) zum Herstellen einer lösbaren Verbindung mit dem Vorsprung (11) der Intraokularlinse (100) ausgebildet. Zuvor wurde die Öffnung (31) im Kapselsack (30) mittels eines Femtosekundenlasers hergestellt.

Der Bereich (30a) des Kapselsacks wurde zur besseren Darstellung der anderen Merkmale in den Figuren 6 bis 8 nicht eingezeichnet. Nach Einbringen der Intraokularlinse (100) wird die Öffnung (34a) zum Einbringen der Intraokularlinse (100) typischerweise wieder mittels des Bereichs (30a) des Kapselsacks (30) bedeckt.

### Bezugszeichenliste

- 100: Intraokularlinse
- 10: Fixiervorrichtung
- 11: Vorsprung
- 11a: freies erstes Ende des Vorsprungs
- 11b: zweites Ende des Vorsprungs
- 11c: Höhe des Vorsprungs
- 11d: Mittelbereich des Vorsprungs
- 12: optischer Bereich
- 12a: radial äußerer Rand des optischen Bereichs
- 13: Randbereich
- 15: optische Linse
- 16: Steg
- 17a, 17b: Haltelemente
- 18a, 18b: Haltearme
- 19a, 19b: Abschnitte des radial äußeren Randes des optischen Bereichs
- 20: Umfangsrichtung
- 21: Normalenrichtung
- 22a: Durchmesser des Vorsprungs im Mittelbereich des Vorsprungs
- 22b: Durchmesser des Vorsprungs am freien ersten Ende des Vorsprungs
- 22c: Durchmesser des Vorsprungs am zweiten Ende des Vorsprungs

- 300: Auge
- 30: Kapselsack
- 30a: Bereich des Kapselsacks
- 31: Ausnehmung zum Herstellen einer lösbaren Verbindung mit dem Vorsprung einer Intraokularlinse
- 31a: Öffnung zum Herstellen einer lösbaren Verbindung mit dem Vorsprung einer Intraokularlinse
- 32: Iris
- 33: Hornhaut
- 34: Ausnehmung zum Einbringen der Intraokularlinse
- 34a: Öffnung zum Einbringen der Intraokularlinse
- 35: natürliche Augenlinse

- 500: Mittel
- 50: erstes Ende des Mittels
- 51: zweites Ende des Mittels
- 52: Griffbereich
- 53: Bereich zum Halten der Intraokularlinse
- 54: erster Bereich des Mittels
- 55: erste Gerade
- 56: zweiter Bereich des Mittels
- 57: zweite Gerade
- 58: Winkel zwischen erster Gerade und zweiter Gerade
- 59: Übergangsbereich zwischen ersten Bereich und zweiten Bereich
- 60: Knick

- 700: Mittel
- 70: erstes Ende des Mittels
- 71: zweites Ende des Mittels
- 72: Griffbereich
- 73: Bereich zum Einführen eines freien Endes des Vorsprungs in die Ausnehmung des Kapselsacks
- 73a: erstes Ende des Bereichs zum Einführen eines freien Endes des Vorsprungs in die Ausnehmung des Kapselsacks
- 73b: zweites Ende des Bereichs zum Einführen eines freien Endes des Vorsprungs in die Ausnehmung des Kapselsacks
- 74: erster Bereich des Mittels
- 75: erste Gerade
- 76: zweiter Bereich des Mittels
- 77: zweite Gerade
- 78: Winkel zwischen erster Gerade und zweiter Gerade 79 Übergangsbereich zwischen erstem Bereich und zweitem Bereich
- 80: Knick
- 81: Dickenrichtung
- 82: Kegelstumpfmantel
- 83: erste Öffnung
- 84: zweite Öffnung
- 85: Höhe des Kegelstumpfes

- 90: Pinzette
- 91: Ultraschallsonde

## Patentansprüche

1. Intraokularlinse (100),
wobei die Intraokularlinse (100) eine Fixiervorrichtung (10) zum Fixieren der Intraokularlinse (100) in einem Kapselsack (30) eines Auges (300) aufweist,
wobei die Fixiervorrichtung (10) einen Vorsprung (11) umfasst,
wobei die Intraokularlinse (100) einen optischen Bereich (12) und einen Randbereich (13) aufweist,
wobei die Fixiervorrichtung (10) im Randbereich (13) der Intraokularlinse (100) angeordnet ist,
wobei der Vorsprung (11) eine erstes, freies Ende (11a), ein zweites, der Intraokularlinse zugewandtes Ende (11b) und einen dazwischen liegenden Mittelbereich (11d) aufweist,
**dadurch gekennzeichnet, dass**
der Vorsprung radialsymmetrisch ausgebildet ist,
wobei sich der Vorsprung (11) ausgehend vom zweiten Ende (11b) im Mittelbereich (11d) verjüngt und sich in Richtung des ersten Endes (11a) erneut erweitert, wobei der Durchmesser des Vorsprungs (11) im Mittelbereich (11d) kleiner ist als der Durchmesser am ersten Ende (11a) und der Durchmesser am zweiten Ende (11b),
und wobei der Durchmesser des Vorsprungs (11) am ersten Ende (11a) größer ist als der Durchmesser am zweiten Ende (11b).

2. Intraokularlinse (100) nach Anspruch 1,
**dadurch kennzeichnet,**
**dass** die Fixiervorrichtung (10) derart ausgebildet ist, dass die Fixiervorrichtung (10) zum Herstellen einer lösbaren Verbindung mit einer korrespondierenden Ausnehmung (31) in dem Kapselsack (30) des Auges (300) geeignet ist.

3. Intraokularlinse (100) nach einem der vorherigen Ansprüche,
**dadurch gekennzeichnet,**
**dass** sich die Fixiervorrichtung (10) im Wesentlichen in Dickenrichtung (14) der Intraokularlinse (100) erstreckt.

4. Intraokularlinse (100) nach einem der vorherigen Ansprüche,
**dadurch gekennzeichnet,**
**dass** der Vorsprung (11) der Intraokularlinse (100) hinterschnitten ausgebildet ist.

5. Intraokularlinse (100) nach einem der vorherigen Ansprüche,
**dadurch gekennzeichnet,**
**dass** die Intraokularlinse (100) torisch ausgebildet ist.

6. Mittel (500) zur Herstellung einer lösbaren Verbindung zwischen einem Vorsprung (11) einer Intraokularlinse (100) nach einem der Ansprüche 1 bis 5 mit einer korrespondierenden Ausnehmung (31) in einem Kapselsack (30) eines Auges (300),
wobei das Mittel (500) zum Halten der Intraokularlinse (100) ausgebildet ist,
wobei das Mittel (500) an einem ersten Ende (50) einen Griffbereich (52) zum Halten des Mittels (500) aufweist,
wobei das Mittel (500) an einem zweiten Ende (51) einen Bereich (53) zum Halten der Intraokularlinse (100) aufweist,
wobei das Mittel (500) zwischen dem ersten Ende (50) und dem zweiten Ende (51) derart ausgebildet ist, dass sich das Mittel (500) ausgehend vom ersten Ende (50) in einem ersten Bereich (54) im Wesentlichen entlang einer ersten Gerade (55) erstreckt und dass sich das Mittel (500) in einem zweiten Bereich (56) im Wesentlichen entlang einer zweiten Gerade (57) erstreckt,
wobei die erste Gerade (55) und die zweite Gerade (57) in einem Winkel (58) zueinander stehen,
wobei der Bereich (53) zum Halten der Intraokularlinse (100) gebogen, insbesondere hakenförmig, ausgebildet ist,
**dadurch gekennzeichnet,**
**dass** die erste Gerade (55) und die zweite Gerade (57) eine erste Ebene bilden,
wobei sich der Bereich (53) zum Halten der Intraokularlinse (100) in einer zweiten Ebene erstreckt, wobei die zweite Ebene die zweite Gerade (57) umfasst und unter einem Winkel zur ersten Ebene ausgebildet ist.

7. Mittel (700) zur Herstellung einer lösbaren Verbindung zwischen einem Vorsprung (11) einer Intraokularlinse (100) nach einem der Ansprüche 1 bis 5 mit einer korrespondierenden Ausnehmung (31) in einem Kapselsack (30) eines Auges (300),
wobei das Mittel (700) zum Einführen eines freien Endes (11a) des Vorsprungs (11) in die Ausnehmung (31) des Kapselsacks (30) ausgebildet ist,
wobei das Mittel (700) an einem ersten Ende (70) einen Griffbereich (72) zum Halten des Mittels (700) aufweist,
wobei das Mittel (700) an einem zweiten Ende (71) einen Bereich (73) zum Einführen eines freien Endes (11a) des Vorsprungs (11) in die Ausnehmung (31) des Kapselsacks (30) aufweist,
wobei der Bereich (73) zum Einführen eines freien Endes (11a) des Vorsprungs (11) in die Ausnehmung (31) des Kapselsacks (30) ringförmig ausgebildet ist, wobei das Mittel (700) zwischen dem ersten Ende (70) und dem zweiten Ende (71) derart ausgebildet ist, dass sich das Mittel (700) ausgehend vom ersten Ende (70) in einem ersten Bereich (74) im Wesentlichen entlang einer ersten Gerade (75) erstreckt und das sich das Mittel (700) in einem zweiten Bereich (76) im Wesentlichen entlang einer zweiten Geraden (77) erstreckt, wobei die erste Gerade (75) und die zweite Gerade (77) in einem Winkel (78) zueinander stehen,
**dadurch gekennzeichnet, dass**
der Bereich (73) zum Einführen eines freien Endes (11a) des Vorsprungs (11) in die Ausnehmung (31) des Kapselsacks (30) kegelstumpfförmig ausgebildet ist.

8. Mittel (700) nach Anspruch 7,
**dadurch gekennzeichnet, dass**
der Winkel (78) zwischen 15° und 75° beträgt, bevorzugt zwischen 20° und 70°, besonders bevorzugt zwischen 30° und 60° und ganz besonders bevorzugt zwischen 40° und 50° beträgt.

9. Mittel (700) nach einem der Ansprüche 7 oder 8,
**dadurch gekennzeichnet, dass** das Mittel (700) in einem Übergangsbereich (59, 79) zwischen dem ersten Bereich (54, 74) und dem zweiten Bereich (56, 76) einen Knick (60, 80) und/oder eine Krümmung aufweist.

## Claims

1. Intraocular lens (100),
wherein the intraocular lens (100)has a fixing device (10) for fixing the intraocular lens (100) in a capsular sac (30) of an eye (300),
wherein the fixing device (10) comprises a protrusion (11),
wherein the intraocular lens (100) comprises an optical region (12) and an edge region (13),
wherein the fixing device (10) is arranged in the edge region (13) of the intraocular lens (100),
wherein the protrusion (11) has a first, free end (11a) and a second end (11b) facing the intraocular lens (100) and a middle portion (11d) arranged between the first end (11a) and the second end (11b),
**characterized in that**
the protrusion is configured radially symmetrical,
wherein the protrusion (11) tapers from the second end (11b) in the middle portion (11d) and widens again towards the first end (11a), wherein the diameter of the protrusion (11) in the middle portion (11d) is smaller than the diameter at the first end (11a) and the diameter at the second end (11b), and wherein the diameter of the protrusion (11) at the first end (11a) is larger than the diameter at the second end (11b).

2. Intraocular lens (100) according to claim 1,
**characterized in that**
the fixing device (10) is configured such that the fixing device (10) is suitable for establishing a detachable connection with a corresponding recess (31) in the capsular sac (30) of the eye (300).

3. Intraocular lens (100) according to any of the preceding claims,
**characterized in that**
the fixing device (10) extends essentially in a thickness direction (14) of the intraocular lens (100).

4. Intraocular lens (100) according to any of the preceding claims,
**characterized in that**
the protrusion (11) of the intraocular lens (100) is configured undercut.

5. Intraocular lens (100) according to any of the preceding claims,
**characterized in that**
the intraocular lens (100) is configured toric.

6. Means (500) for establishing a detachable connection between a protrusion (11) and an intraocular lens (100) according to any of the claims 1 to 5 with a corresponding recess (31) in a capsule sac (30) of an eye (300),
wherein the means (500) is configured to hold the intraocular lens (100),
wherein the means (500) comprises a gripping portion (52) for holding the means (500) at a first end (50),
wherein the means (500) comprises a portion (53) at a second end (51) for holding the intraocular lens (100),
wherein the means (500) between the first end (50) and the second end (51) is configured such that the means (500) extends from the first end (50) essentially along a first straight line (55) in a first portion (54) and that the means (500) extends essentially along a second straight line (57) in a second portion (56),
wherein the first straight line (55) and the second straight line (57) are positioned at an angle (58) to one another,
wherein the portion (53) for holding the intraocular lens (100) is configured bent, in particular hook-shaped,
**characterized in that**
the first straight line (55) and the second straight line (57) form a first plane,
wherein the portion (53) for holding the intraocular lens (100) extends in a second plane, wherein the second plane comprises the second straight line (57) and is configured at an angle relative to the first plane.

7. Means (700) for establishing a detachable connection between a protrusion (11) of an intraocular lens (100) according to any of the claims 1 to 5 with a corresponding recess (31) in a capsule sac (30) of an eye (300),
wherein the means (700) is configured for insertion of a free and (11a) of the protrusion (11) into the recess (31) of the capsule sac (30),
wherein the means (700) comprises a gripping portion (72) for holding the means (700) at a first end (70),
wherein the means (700) comprises a portion (73) for insertion of a free end (11a) of the protrusion (11) into the recess (31) of the capsule sac (30) at a second end (71),
wherein the portion (73) is configured ring-shaped for insertion of a free end (11a) of the protrusion (11) into the recess (31) of the capsule sac (30),
wherein the means (700) between the first end (70) and the second end (71) is configured such that the means (700) extends from the first end (70) essentially along a first straight line (75) in a first portion (74) and that the means (700) extends essentially along a second straight line (77) in a second portion (76), wherein the first straight line (75) and the second straight line (77) are positioned at an angle (78) to one another,
**characterized in that**
the portion (73) for insertion of a free end (11a) of the protrusion (11) into the recess (31) of the capsule sac (30) is configured in a truncated cone-shaped manner.

8. Means (700) according to claim 7,
**characterized in that**
the angle (78) is between 15° and 75°, preferably between 20° and 70°, particularly preferably between 30° and 60°, and most preferably between 40° and 50°.

9. Means (700) according to one of claims 7 or 8,
**characterized in that**
the means (700) comprises a kink (60, 80) and/or a curvature in a transition portion (59, 79) between the first portion (54,74) and the second portion (56, 76).

## Revendications

1. Lentille intraoculaire (100),
dans laquelle la lentille intraoculaire (100) présente un dispositif de fixation (10) pour fixer la lentille intraoculaire (100) dans un sac capsulaire (30) d'un œil (300),
dans laquelle le dispositif de fixation (10) comprend une saillie (11),
dans laquelle la lentille intraoculaire (100) présente une zone optique (12) et une zone marginale (13),
dans laquelle le dispositif de fixation (10) est disposé dans la zone marginale (13) de la lentille intraoculaire (100),
dans laquelle la saillie (11) présente une première extrémité libre (11a), une deuxième extrémité (11b) tournée vers la lentille intraoculaire, et une zone centrale (11d) située entre les deux,
**caractérisée en ce que** la saillie est réalisée selon une symétrie radiale,
dans laquelle la saillie (11) s'amincit en partant de la deuxième extrémité (11b) dans la zone centrale (11d) et s'élargit de nouveau en direction de la première extrémité (11a), le diamètre de la saillie (11) dans la zone centrale (11d) étant inférieur au diamètre à la première extrémité (11a) et au diamètre à la deuxième extrémité (11b),
et dans laquelle le diamètre de la saillie (11) à la première extrémité (11a) est supérieur au diamètre à la deuxième extrémité (11b).

2. Lentille intraoculaire (100) selon la revendication 1, **caractérisée en ce que** le dispositif de fixation (10) est réalisé de telle sorte que le dispositif de fixation (10) est adapté pour établir une connexion amovible avec un évidement correspondant (31) dans le sac capsulaire (30) de l'œil (300).

3. Lentille intraoculaire (100) selon l'une quelconque des revendications précédentes, **caractérisée en ce que** le dispositif de fixation (10) s'étend substantiellement dans la direction de l'épaisseur (14) de la lentille intraoculaire (100).

4. Lentille intraoculaire (100) selon l'une quelconque des revendications précédentes, **caractérisée en ce que** la saillie (11) de la lentille intraoculaire (100) est réalisée en contre-dépouille.

5. Lentille intraoculaire (100) selon l'une quelconque des revendications précédentes, **caractérisée en ce que** la lentille intraoculaire (100) est réalisée de manière torique.

6. Moyen (500) permettant d'établir une connexion amovible entre une saillie (11) d'une lentille intraoculaire (100) selon l'une quelconque des revendications 1 à 5 avec un évidement correspondant (31) dans un sac capsulaire (30) d'un œil (300),
dans lequel le moyen (500) est réalisé pour tenir la lentille intraoculaire (100), dans lequel le moyen (500) présente à une première extrémité (50) une zone de préhension (52) pour tenir le moyen (500),
dans lequel le moyen (500) présente à une deuxième extrémité (51) une zone (53) pour tenir la lentille intraoculaire (100),
dans lequel le moyen (500) est réalisé entre la première extrémité (50) et la deuxième extrémité (51) de telle sorte que le moyen (500) s'étend à partir de la première extrémité (50) dans une première zone (54) substantiellement le long d'une première ligne droite (55) et en ce que le moyen (500) s'étend dans une deuxième zone (56) substantiellement le long d'une deuxième ligne droite (57), dans lequel la première ligne droite (55) et la deuxième ligne droite (57) forment un angle (58) l'une par rapport à l'autre,
dans lequel la zone (53) pour tenir la lentille intraoculaire (100) est réalisée de manière recourbée, en particulier en forme de crochet,
**caractérisé en ce que** la première ligne droite (55) et la deuxième ligne droite (57) forment un premier plan, la zone (53) pourtenir la lentille intraoculaire (100) s'étendant dans un deuxième plan, le deuxième plan comprenant la deuxième ligne droite (57) et étant réalisé selon un angle par rapport au premier plan.

7. Moyen (700) permettant d'établir une connexion amovible entre une saillie (11) d'une lentille intraoculaire (100) selon l'une quelconque des revendications 1 à 5 avec un évidement correspondant (31) dans un sac capsulaire (30) d'un œil (300),
dans lequel le moyen (700) est réalisé pour introduire une extrémité libre (11a) de la saillie (11) dans l'évidement (31) du sac capsulaire (30),
dans lequel le moyen (700) présente à une première extrémité (70) une zone de préhension (72) pour tenir le moyen (700),
dans lequel le moyen (700) présente à une deuxième extrémité (71) une zone (73) pour introduire une extrémité libre (11a) de la saillie (11) dans l'évidement (31) du sac capsulaire (30),
dans lequel la zone (73) pour introduire une extrémité libre (11a) de la saillie (11)
dans l'évidement (31) du sac capsulaire (30) est réalisée en forme d'anneau,
dans lequel le moyen (700) est réalisé entre la première extrémité (70) et la deuxième extrémité (71) de telle sorte, que le moyen (700) s'étend en partant de la première extrémité (70) dans une première zone (74) substantiellement le long d'une première ligne droite (75) et en ce que le moyen (700) s'étend dans une deuxième zone (76) substantiellement le long d'une deuxième ligne droite (77), la première ligne droite (75) et la deuxième ligne droite (77) formant un angle (78) l'une par rapport à l'autre,
**caractérisé en ce que** la zone (73) pour introduire une extrémité libre (11a) de la saillie (11) dans l'évidement (31) du sac capsulaire (30) est réalisée en forme de cône tronqué.

8. Moyen (700) selon la revendication 7, **caractérisé en ce que** l'angle (78) est compris entre 15° et 75°, de préférence entre 20° et 70°, de plus grande préférence entre 30° et 60° et de la plus grande préférence entre 40° et 50°.

9. Moyen (700) selon la revendication 7 ou 8, **caractérisé en ce que** le moyen (700) présente dans une zone de transition (59, 79) entre la première zone (54, 74) et la deuxième zone (56, 76) un coude (60, 80) et/ou une courbure.
